# EUROPÄISCHE PATENTANMELDUNG

(11) **EP 1 882 481 A1**
(43) Veröffentlichungstag der Anmeldung: **30.01.2008**
(21) Anmeldenummer: 07012978.8
(22) Anmeldetag: 03.07.2007
(51) Int. Cl.: A61L 9/14

(54) **Verfahren und Vorrichtungen zum Desodorieren**

(30) Priorität: 27.07.2006 DE 102006034694
(71) Anmelder: Air & D- Sarl, 67190 Rosheim (FR)
(72) Erfinder: Janin, Jean-Paul, 69480 Lachassagne (FR); Wuest, Robert, 67190 Rosheim (FR)
(74) Vertreter: Wagner, Jutta

(57) **Zusammenfassung**

Die Erfindung betrifft ein Verfahren und Vorrichtungen zum Desodorieren *bzw. zum Beduften* von geschlossenen Räumen oder großflächigen Anlagen, in denen sich übelriechende Substanzen befinden, durch Behandeln der verunreinigten Luft mit flüchtigen, aktiven Agenzien, die mittels Druckluft durch eine oder mehrere Düsen an die Umgebungsluft zerstäubt bzw. versprüht werden.

## Beschreibung

Die Erfindung betrifft ein Verfahren und Vorrichtungen zum Desodorieren von geschlossenen Räumen oder großflächigen Anlagen, in denen sich übelriechende, flüchtige Substanzen befinden bzw. freigesetzt werden, durch Behandeln der verunreinigten Luft mit flüchtigen, aktiven Agenzien, die mit den übelriechenden Substanzen reagieren bzw. diese maskieren.

In der EP-C 1 471 984 ist ein Verfahren zum Entfernen oder Maskieren von übelriechenden Substanzen in geschlossenen Räumen durch Reaktion mit aktiven Agenzien beschrieben. Diese sind an einem gelartigen, hydrophile Gruppen enthaltenden Polymeren angequollen, mit dem sie zusammen eine schwammartige Struktur bilden, und aus dem sie langsam freigesetzt werden und mit den übelriechenden Substanzen in der Luft reagieren.
In der WO 2004/045657 ist ein Verfahren zum Desodorieren von großflächigen Anlagen, in denen übelriechende Stoffe gelagert sind, durch Reaktion mit aktiven Agenzien beschrieben. Diese sind in einem gelartigen, hydrophile Gruppen enthaltenden Polymeren verteilt, mit dem sie zusammen eine schwammartige Masse bilden. Diese ist zwischen zwei Platten eingelagert, die über der Oberfläche der übelriechenden Stoffe angebracht sind. Durch einen Luftstrom, der zwischen den parallelen Platten hindurch die schwammartige Masse bestreicht, werden die aktiven Agenzien freigesetzt.

Beide Verfahren haben den Nachteil, dass erst das hydrophile Gruppen tragende Polymere hergestellt und mit den aktiven Agenzien zusammen zu der schwammartigen Masse beladen werden muss.

Der Erfindung lag also die Aufgabe zugrunde, diesen Nachteil zu vermeiden und ein einfaches Verfahren zum Desodorieren bereitzustellen.

Es wurde nun gefunden, dass diese Aufgabe gelöst wird, wenn die flüchtigen aktiven Agenzien mittels Druckluft durch eine oder mehrere Düsen mit Hilfe des Venturi- Prinzips an die Umgebungsluft zerstäubt bzw. versprüht werden.

Das Prinzip der Venturi- Düse ist an sich bekannt. Wenn die Düse von Gasen, zum Beispiel Druckluft, durchströmt wird, verändert sich der im Rohr herrschende Druck. An der engsten Stelle des Rohres ist der Staudruck maximal und der Ruhedruck minimal. Die Geschwindigkeit der fließenden Luft steigt im Verhältnis der Querschnitte beim Durchströmen des eingeschnürten Teils an, weil überall die gleiche Menge durchfließt. Gleichzeitig sinkt der Druck im Abnahmerohr, das im engen Teil sitzt. Damit entsteht ein Vakuum, dass zum Ansaugen von Gasen und Flüssigkeiten benutzt werden kann.

Dieses Prinzip wird bei der vorliegenden Erfindung benutzt, um aktive Substanzen aus einem Vorratsbehälter anzusaugen und zu zerstäuben bzw. zu versprühen. Dabei entstehen je nach dem Druck der in die Düse eingepressten Luft, der zwischen *1,05* und 8 bar, *vorzugsweise zwischen 2 und 4 bar* liegen kann, Partikel mit einem Durchmesser zwischen 0,1 und 10 µm, vorzugsweise zwischen 0,2 und 5 µm.
Bei einer bevorzugten Ausführungsform der Erfindung sinken die gröberen Bestandteile, z.B. mit einem Durchmesser von mehr als 5 µm, nach dem Austritt an die Umgebungsluft wieder ab und können in den Vorratsbehälter zurückfließen.

Bei den übelriechenden Substanzen handelt es sich vor allem um anorganische und organische Stickstoff- und Schwefelverbindungen, insbesondere um Ammoniak und Schwefelwasserstoff; daneben auch um Phenole, Halogen- und Kohlenwasserstoff-Verbindungen. Sie sind entweder in der Umgebungsluft enthalten oder werden von festen oder flüssigen Stoffen, insbesondere in großflächigen Anlagen, freigesetzt. Geeignete aktive Agenzien sind Aldehyde, Ketone, Alkohole und Ester, beispielsweise Vanillin, Eugenol, Thymol, Geraniol, Kampferöl, Citronellol, Linanol, Menthol, Cumarin, Citral, Alpha-Pinen, Nerylacetat, Linalylacetat, Butylhydroxytoluol, C7- bis C12- Aldehyde, Salicylbenzylester und natürliche ölige Essenzen.

Es handelt sich dabei um Flüssigkeiten oder Feststoffe mit verhältnismäßig hohem Siedepunkt, die aber - wenn sie in sehr feiner Verteilung vorliegen - flüchtig sind und zerstäubt werden können. Falls erforderlich, können sie auch mit Lösungsmitteln, z.B. mit Alkoholen oder mit pflanzlichen Ölen verdünnt *oder in Wasser emulgiert werden.*

Die aktiven Agenzien können mit den übelriechenden Substanzen eine chemische Reaktion eingehen und sie dadurch *neutralisieren* oder zumindest reduzieren. Daneben kann es auch zu Bindungen durch elektrostatische oder van der Waals'sche Kräfte kommen, wodurch die Geruchswahrnehmbarkeit zumindest herabgesetzt wird. Schließlich können die aktiven Substanzen zusätzlich als Parfüme wirken und dadurch die übelriechenden Substanzen maskieren.

Ein wesentlicher Vorteil des erfindungsgemäßen Verfahrens ist, dass durch eine geeignete Steuerung, z.B. durch einen Timer, die Erzeugung der Druckluft mittels eines Kompressors angestellt bzw. abgestellt oder in zeitlichen Sequenzen aktiviert werden kann. Damit wird es möglich, die aktiven Agenzien nur dann zu zerstäuben, wenn es erforderlich ist.

Das erfindungsgemäße Verfahren kann zum Desodorieren von geschlossenen Räumen angewandt werden, insbesondere dort, wo der durch die zerstäubten Agenzien gebildete Nebel nicht störend ist. Beispiele sind Müllfahrzeuge, Toiletten, Tierställe, Abluftschächte, Ventilationskästen sowie stark frequentierte Büroräume. *Eine besonders interessante Anwendung ist die Parfümierung von Flughafenhallen.*

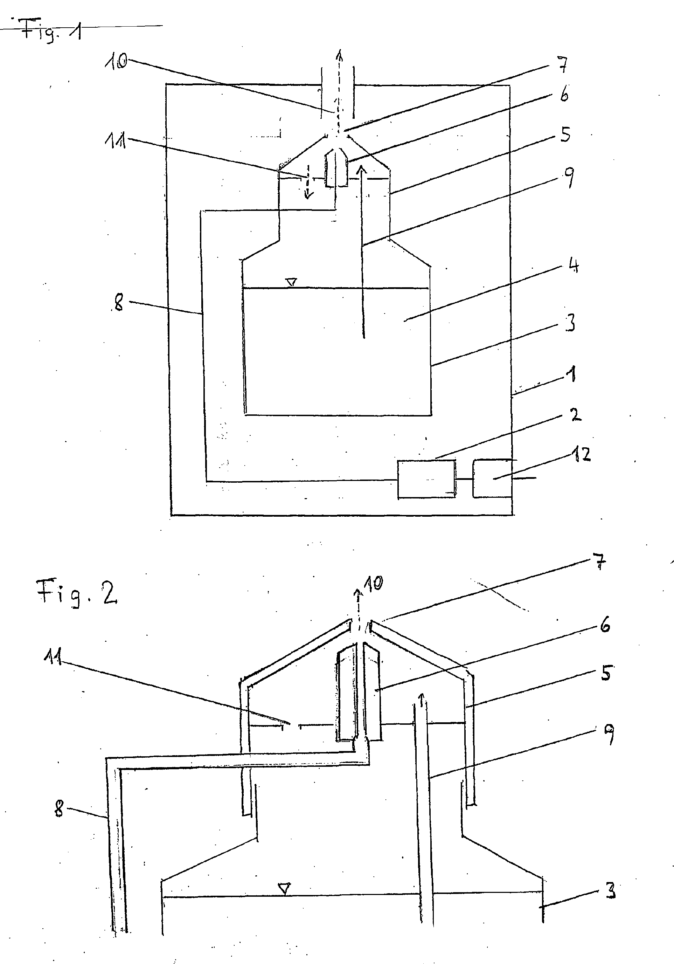

Eine geeignete Vorrichtung ist in Figur 1 schematisch dargestellt. Sie besteht aus einem Kasten (1), dessen Größe je nach Anwendung 100 ml bis 10 l, vorzugsweise 200 ml bis 1 l betragen kann. In seinem Inneren befinden sich folgende Einbauten:
- ein Kompressor (2) zur Erzeugung von Druckluft, vorzugsweise mit einem Druck von 1,2 bis 2,4 bar, insbesondere von 1,4 bis 1,8 bar,
- ein Behälter (3) mit den flüchtigen aktiven Agenzien (4), vorzugsweise aus Stahlblech,
- ein Aufsatz (5) auf dem Behälter mit der Düse (6) und einer Öffnung (7) an seinem oberen Ende zum Zerstäuben bzw. Versprühen der aktiven Agenzien an die Umgebungsluft,
- ein Rohr oder Schlauch (8), der den Kompressor mit der Düse verbindet - und die Druckluft dort einleitet, sowie
- ein Rohr (9) zum Ansaugen der aktiven Agenzien aus dem Behälter.

Durch den von der Düse (6) erzeugten Unterdruck werden aus dem Behälter (3) die aktiven Agenzien durch das Rohr (9) angesaugt und durch die Öffnung (7) an die Umgebungsluft zerstäubt (10).
Die obere Öffnung der Düse (6), aus der die Druckluft ausströmt, ist vorzugsweise zwischen 0,4 und 0,8 mm breit, die Öffnung (7) des Behälters ist vorzugsweise 0,8 bis 1,2 mm breit. Es wird angenommen, dass diese Differenz den Unterdruck bewirkt, durch den die aktiven Agenzien angesaugt werden.

Bei einer bevorzugten Ausführungsform der Erfindung befinden sich im Aufsatz (5) Öffnungen (11), durch welche die abgesunkenen groben Bestandteile der zerstäubten aktiven Agenzien wieder in den Behälter zurückfließen können.

Bei einer weiteren bevorzugten Ausführungsform befindet sich in dem Kasten (1) zusätzlich ein Steuergerät (12), z.B. ein Timer, durch welchen die Aktivität des Kompressors in zeitlichen Sequenzen gesteuert werden kann.

Figur 2 zeigt eine für die vorliegende Erfindung zweckmäßige Ausgestaltung des oberen Teils der Vorrichtung, wobei die Bezeichnungen die selbe Bedeutung wie in Figur 1 haben. Selbstverständlich sind Abwandlungen der in den Figuren dargestellten Ausgestaltungen möglich.

Das erfindungsgemäße Verfahren kann auch zum Desodorieren von vorzugsweise offenen, großflächigen Anlagen benutzt werden, beispielsweise für Klärbecken, Kläranlagen, Kompostieranlagen, Mülldeponien, Tierzuchtanlagen, Abwasser-stapelbecken, Hafenbecken und Abwasserkanäle sowie Großindustrieanlagen mit verschiedenartigen Emissionen.

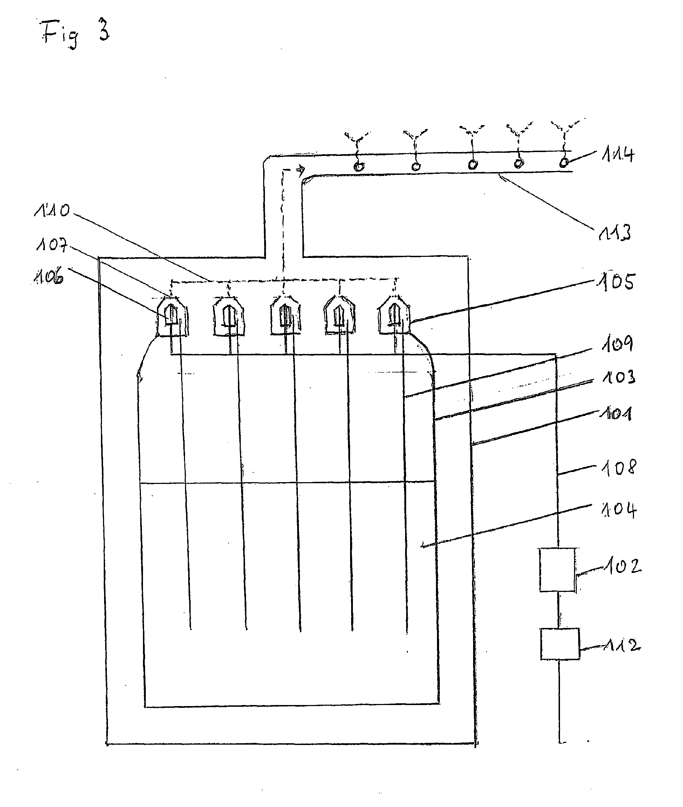

Eine dafür geeignete Vorrichtung ist in Figur 3 schematisch dargestellt. Sie *ist vorzugsweise wieder in* einem Kasten (101) *untergebracht,* dessen Größe je nach Anwendung 10 bis 200 I, vorzugsweise 50 bis 100 I betragen kann. *Die Vorrichtung umfasst* folgende Einbauten:
- einen Behälter (103) mit den flüchtigen Agenzien (104), vorzugsweise in Form eines Fasses aus Stahlblech,
- eine Vielzahl, vorzugsweise 2 bis 40, von Aufsätzen (105) mit Düsen (106), die z.B. ringförmig auf dem Behälter angeordnet sind,
- ein Rohr oder *einen* Schlauch (108), *die* einen vorzugsweise außerhalb des Kastens befindlichen Kompressor (112) mit den Düsen verbindet und die Druckluft, vorzugsweise mit einem Druck von 1,3 bis 2,3 bar in die einzelnen Düsen einleitet,
- Rohre (109) zum Ansaugen der aktiven Agenzien aus dem Behälter, sowie außerhalb des Kastens
- einen steifen oder flexiblen Schlauch (113), in dem die aus den Öffnungen austretenden aktiven Agenzien gesammelt werden, mit Löchern (114), aus denen die aktiven Agenzien an die Umgebungsluft zerstäubt bzw. versprüht werden.

Bei einer bevorzugten Ausführungsform der Erfindung ist auch hier ein Steuergerät (112) vorgesehen, durch welches die Aktivität des Kompressors (102) in zeitlichen Sequenzen gesteuert werden kann. *Ferner kann ein Druckregler zur Einstellung des optimalen Drucks der einzuleitenden Druckluft sowie ein Elektroventil zum Ein- und Ausschalten der Druckluft hinter dem Kompressor angebracht sein.*

## Patentansprüche

1. Verfahren zum Desodorieren von geschlossenen Räumen oder großflächigen Anlagen, in denen sich übelriechende, flüchtige Substanzen befinden bzw. freigesetzt werden, durch Behandeln der verunreinigten Luft mit flüchtigen, aktiven Agenzien, die mit den übelriechenden Substanzen reagieren bzw. diese maskieren, **dadurch gekennzeichnet, dass** die flüchtigen Agenzien mittels Druckluft durch eine oder mehrere Düsen an die Umgebungsluft zerstäubt bzw. versprüht werden, wobei sie mit Hilfe des Venturi- Effekts durch Unterdruck aus einem oder mehreren Behältern herausgesaugt wurden.

2. Verfahren nach Anspruch 1, **dadurch gekennzeichnet, dass** man bei dem Versprühen Partikel mit einem Durchmesser von 0,1 bis 10 µm erzeugt, von denen die gröberen Bestandteile wieder absinken und in den Behälter zurückfließen.

3. Verfahren nach Anspruch 1, **dadurch gekennzeichnet, dass** die aktiven Agenzien Aldehyde, Ketone, Alkohole oder Ester sind.

4. Vorrichtung zum Desodorieren von geschlossenen Räumen nach Anspruch **1, dadurch gekennzeichnet, dass** in einem Kasten (1) folgende Einbauten angeordnet sind:
- ein Kompressor (2) zur Erzeugung von Druckluft,
- ein Behälter (3) mit den flüchtigen, aktiven Agenzien (4),
- ein Aufsatz (5) auf dem Behälter mit der Düse (6) und einer Öffnung (7) an seinem oberen Ende zum Zerstäuben bzw. Versprühen der aktiven Agenzien an die Umgebungsluft,
- ein Rohr oder Schlauch (8), der den Kompressor mit der Düse verbindet und die Druckluft dort einleitet, sowie
- ein Rohr (9) zum Ansaugen der aktiven Agenzien aus dem Behälter.

5. Vorrichtung nach Anspruch 4, **dadurch gekennzeichnet, dass** sich in dem Aufsatz (5) Öffnungen (11) befinden, durch welche die abgesunkenen groben Bestandteile der zerstäubten aktiven Agenzien wieder in den Behälter (3) zurückfließen können.

6. Vorrichtung nach Anspruch 4, **dadurch gekennzeichnet, dass** sich in dem Kasten (1) zusätzlich ein Steuergerät (12) befindet, durch welches die Aktivität des Kompressors (2) in zeitlichen Sequenzen gesteuert werden kann

7. Vorrichtung zum Desodorieren von großflächigen Anlagen nach Anspruch 1, **dadurch gekennzeichnet, dass** *sie* folgende Einbauten *umfasst, die gegebenenfalls in einem Kasten (101) angeordnet sind:*
- einen Behälter (103) mit den flüchtigen Agenzien (104),
- eine Vielzahl von Aufsätzen (105) auf dem Behälter mit Düsen (106), die vorzugsweise ringförmig auf dem Behälter (103) angeordnet sind, und Öffnungen (107) am oberen Ende der Aufsätze,
- ein Rohr oder *einen* Schlauch (108), der einen vorzugsweise außerhalb des Kastens befindlichen Kompressor (102) mit den Düsen verbindet und die Druckluft in diese einleitet,
- Rohre (109) zum Ansaugen der aktiven Agenzien aus dem Behälter, sowie gegebenenfalls außerhalb des Kastens (101)
- einen Schlauch (113), in dem die aus den Öffnungen austretenden aktiven Agenzien gesammelt werden, mit Löchern (114), aus denen die aktiven Agenzien an die Umgebungsluft zerstäubt bzw. versprüht werden.

8. Vorrichtung nach Anspruch 7, **dadurch gekennzeichnet, dass** sich vorzugsweise außerhalb des Kastens (101) zusätzlich ein Steuergerät (112) befindet, durch welches die Aktivität des Kompressors (102) in zeitlichen Sequenzen gesteuert werden kann, *sowie ein Druckregler zur Einstellung des optimalen Drucks der einzuleitenden Druckluft.*
